# EUROPEAN PATENT APPLICATION

(11) **EP 4 593 026 A1**
(43) Date of publication of application: **30.07.2025**
(21) Application number: 23894843.4
(22) Date of filing: 27.10.2023
(51) Int. Cl.: G16H 30/40, G16H 30/20, G06V 10/82, G06V 10/75, A61B 5/055, A61B 6/03, G06N 3/08

(54) **METHOD FOR CONVERTING MEDICAL IMAGES BY MEANS OF ARTIFICIAL INTELLIGENCE WITH IMPROVED VERSATILITY AND DEVICE THEREFOR**

(30) Priority: 23.11.2022 KR 20220158643
(71) Applicant: Polestar Healthcare Co., Ltd., Seoul 06627 (KR)
(72) Inventor: YOON, Yeo Dong, Seoul 06629 (KR); LEE, Eun Chong, Yongin-si Gyeonggi-do 16812 (KR)
(74) Representative: RGTH
(86) International application number: PCT/KR2023/016874
(87) International publication number: WO 2024/111914

(57) **Abstract**

An aspect of the present invention relates to a method for converting medical images by means of artificial intelligence and, more specifically, to a method for converting medical images by using a generative adversarial network (GAN) with improved versatility and a device therefor. An embodiment of the present invention may provide a medical image conversion system wherein, in order for a learning model to exhibit a predetermined level of performance with regard to operations required by various institutions, various evaluations/opinions from expert groups belonging to various institutions are reflected, thereby improving model versatility.

## Description

### TECHNICAL FIELD

An aspect of the disclosure relates to a method of converting a medical image using artificial intelligence (Al) and, more particularly, to an image conversion method and device for converting a medical image using a generative adversarial network (GAN) with improved versatility.

### BACKGROUND ART

The information disclosed in this section is only provided for an understanding of background information of embodiments of the disclosure and should not be taken as a description of the prior art.

For a medical image conversion system using a learning model which performs machine learning, it is important to collect training data that serves as the basis for the training.

Training data based on medical data is usually collected from hospitals, institutions, and the like, and may be significantly difficult to access due to security and other requirements for personal information.

Therefore, it is common for information to be obtained for use as training data only from a particular affiliated hospital or institution.

However, a learning model trained using training data processed by receiving medical information from only a particular hospital or institution is bound to be biased. In other words, an Al model dependent on a particular institution inevitably provides inaccurate outputs for input images provided by other hospitals or institutions.

Therefore, it is urgent to develop medical image conversion software using an Al learning model with model universality that may be used in a variety of hospitals or institutions.

The information disclosed in the Background section is technical information that the inventors possessed for, or acquired during, derivation of embodiments of the disclosure and should not be taken as known technology disclosed to the public before the filing of the embodiments of the disclosure.

### DISCLOSURE

### Technical Problem

Accordingly, an aspect of the disclosure has been made to solve the above-described problems, and an objective of the disclosure is to provide a system for converting a medical image which has improved model universality by reflecting various evaluation opinions of a group of experts belonging to various institutions so that a learning model achieves consistent performance for tasks required by various institutions.

Another objective of the disclosure is to provide a system for converting a medical image which enables medical professionals to perform medical examination, diagnosis, and treatment with accurate information and patients to receive appropriate medical services.

The objectives of the disclosure are not limited to the foregoing description, and other objectives not explicitly disclosed herein will be clearly understood by a person having ordinary knowledge in the art to which the disclosure pertains from the description provided hereinafter.

### Technical Solution

In order to achieve at least one of the above objectives, an aspect of the disclosure provides an artificial intelligence (AI) training method for improving versatility of a learning model which is trained using a generative adversarial network (GAN), receives input data and generates an output image for medical image conversion, and compares the output image with a target image to improve accuracy of a result of the medical image conversion, the Al training method including:
a first operation of providing a result of medical image conversion to an evaluator group;
a second operation of receiving an evaluation result corresponding to the result of the medical image conversion from the evaluator group;
a third operation of analyzing evaluation tendency differences of the evaluator group based on the evaluation result; and
a fourth operation of removing the evaluation tendency differences from the evaluation result to generate an unbiased evaluation result and reflecting the unbiased evaluation result in the learning model to train the learning model.

The third operation may include analyzing a first evaluation tendency difference between different affiliated institutions and analyzing a second evaluation tendency difference between different evaluators.

The different evaluators may belong to a single institution.

The third operation may analyze the evaluation tendency differences by a statistical method. In some embodiments, the third operation may analyze the evaluation tendency differences collected over a selected period of time or a selected number of times.

Another aspect of the disclosure provides a device for converting a medical image which is trained using a generative adversarial network (GAN), and receives input data and generates an output image for medical image conversion, the device including:
an evaluator information provider configured to provide a result of medical image conversion to an evaluator group;
an evaluator information receiver configured to receive an evaluation result corresponding to the result of the medical image conversion from the evaluator group;
a difference analyzer configured to analyze evaluation tendency differences of the evaluator group based on the evaluation result; and
a learning part configured to remove the evaluation tendency differences from the evaluation result to generate an unbiased evaluation result and reflect the unbiased evaluation result to a learning model to train the learning model.

The difference analyzer may include a first difference analyzer configured to analyze a first evaluation tendency difference between different institutions and a second difference analyzer configured to analyze a second evaluation tendency difference between different evaluators. In some embodiments, the different evaluators may belong to a single institution.

The difference analyzer may analyze the evaluation tendency differences by a statistical method.

The difference analyzer may analyze the evaluation tendency differences collected over a selected period of time or a selected number of times.

Another aspect of the disclosure provides a method of improving a conversion result of a learning model which is trained using a generative adversarial network (GAN) and performs a medical image conversion, the method including:
an opinion reception operation of providing a result of medical image conversion to an evaluator group and receiving evaluation opinions about the result of the medical image conversion from the evaluator group;
a difference extraction operation of extracting evaluation tendency differences of the evaluator group based on the evaluation opinions; and
an opinion preparation operation of removing the extracted evaluation tendency differences from the evaluation opinions to prepare an unbiased evaluation opinion from which the evaluation tendency differences are removed.

Another aspect of the disclosure provides a system for converting a medical image, the system including: a learning model configured to receive an input image and generate an output image belonging to a different domain from the input image based on the input image;
a first feedback part configured to compare the output image with a target image and feed a result of the comparison back to the learning model; and
a second feedback part configured to provide the output image to evaluators, receive evaluation opinions of the evaluators, extract evaluation tendency differences of the evaluators, remove the evaluation tendency differences from the evaluation opinions to derive an unbiased evaluation opinion from which the evaluation tendency differences are removed, and feed the unbiased evaluation opinion back to the learning model.

### Advantageous Effects

According to an embodiment of the disclosure, a system for converting a medical image may have improved model universality by reflecting various evaluation opinions of a group of experts belonging to various institutions so that a learning model achieves consistent performance for tasks required by various institutions.

According to another embodiment of the disclosure, a system for converting a medical image may enable medical professionals to perform medical examination, diagnosis, and treatment with accurate information and patients to receive appropriate medical services.

As an effect, provided is a method of converting a medical image which enables medical professionals to perform medical examination, diagnosis, and treatment with accurate information and patients to receive appropriate medical services.

In addition, the disclosure has a variety of effects with excellent versatility according to embodiments, and such effects may be clearly understood from the following description of embodiments.

### DESCRIPTION OF DRAWINGS

The following drawings accompanying the specification illustrate embodiments of the disclosure and, together with the foregoing disclosure, serve to provide further understanding of the technical spirit of the disclosure, and thus, the disclosure should not be construed as being limited to the drawings, wherein:
FIG. 1 illustrates a method of converting a medical image according to an embodiment of the disclosure.
FIG. 2 illustrates an Al learning model according to an embodiment of the disclosure.
FIG. 3 illustrates an embodiment of calculating the match rate between an output image and a target image.
FIG. 4 illustrates an Al training method according to another embodiment of the disclosure.
FIG. 5 illustrates a device for converting a medical image according to another embodiment of the disclosure.
FIG. 6 illustrates a method of preprocessing training data according to an embodiment of the disclosure.
FIG. 7 illustrates a system for converting a medical image according to an embodiment of the disclosure.

### BEST MODE

Advantages and features of the disclosure, as well as methods of realizing the same, will be more clearly understood from the following detailed description of embodiments when taken in conjunction with the accompanying drawings. However, the disclosure is not limited to specific embodiments to be described hereinafter but should be understood as including a variety of modifications, equivalents, and alternatives within the spirit and scope of the disclosure. Rather, these embodiments are provided so that the description of the disclosure will be complete and will fully convey the scope of the disclosure to a person having ordinary skill in the art in the technical field to which the disclosure pertains. In the following description of the disclosure, a detailed description of related known technology will be omitted when the description may render the subject matter of the disclosure unclear.

The terminology used herein is for the purpose of describing particular embodiments only and is not intended to be limiting. As used herein, the singular forms are intended to include the plural forms as well, unless the context clearly indicates otherwise.

Terms, such as "comprise/include" or "have," or the like, as used herein, indicate that a feature, a number, a step, an operation, a component, a part or a combination thereof described in the disclosure is present, but do not preclude the possibility of the presence or addition of one or more other features, numbers, steps, operations, components, parts or combinations thereof in advance. Although terms, such as "first", "second", or the like, may be used to describe various components, these components should not be conceived of as being limited by these terms. These terms are only used to distinguish a component from another component.

Hereinafter, embodiments according to the disclosure will be described in detail with reference to the accompanying drawings, in which identical or similar components are given the same reference numerals, and repeated descriptions thereof will be omitted.

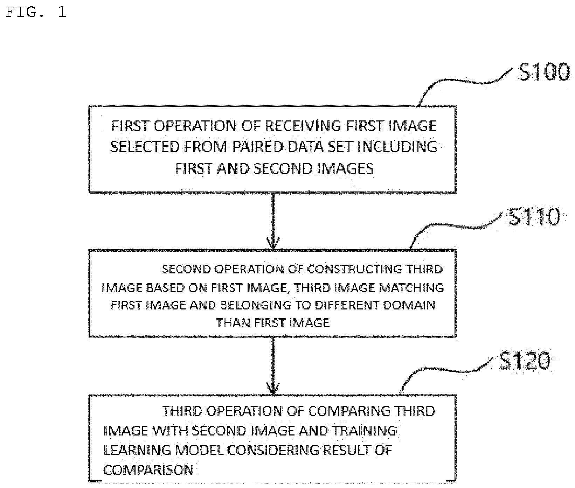

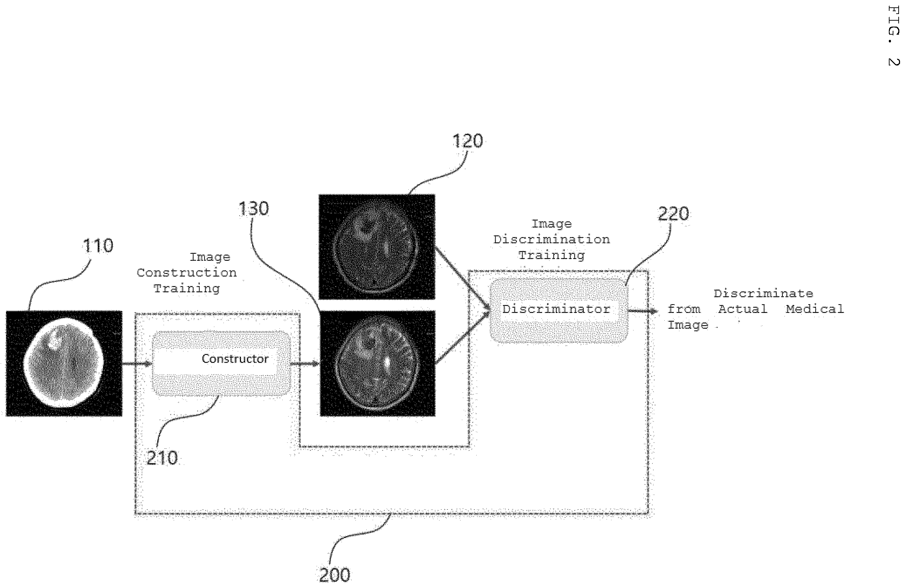

FIG. 1 illustrates a method of converting a medical image according to an embodiment of the disclosure, and FIG. 2 illustrates an artificial intelligence (AI) learning model according to an embodiment of the disclosure.

According to an embodiment of the disclosure, a method of converting a medical image using a generative adversarial network (GAN) may be provided.

The method of converting a medical image according to this embodiment may include: a first operation S100 of receiving a first image 110 selected from a paired data set including the first image 110 and a second image 120; a second operation S110 of constructing a third image 130 based on the first image 110, the third image 130 matching the first image 110 and belonging to a different domain from the first image 110;
a third operation S120 of comparing the third image 130 with the second image 120 and training the learning model considering a result of the comparison; and a fourth operation S130 of comparing the third image 130 with the first image 110 and training the learning model considering a result of the comparison.

The first image 110, the second image 120, and the third image 130 may be medical images. The medical images may be magnetic resonance images, such as 2D MR, 3D MR, 2D streaming MR, 4D MR, 4D volumetric MR, and 4D cine MR images; functional MR images, such as fMR, DCE-MR, and diffusion MR images; computed tomography (CT) images, such as 2D CT, cone beam CT, 3D CT, and 4D CT images; ultrasound images, such as 2D ultrasound, 3D ultrasound, and 4D ultrasound images; positron emission tomography (PET) images; X-ray images; fluoroscopic images; radiotherapy portal images; single-photon emission computed tomography (SPECT) images; computer generated synthetic images, such as pseudo-CT images; and the like.

Furthermore, the medical images may include medical image data, such as a training image, a ground truth image, a contoured image, a dose image, and the like.

In some embodiments, the medical image may include an image acquired using an image acquisition device, a computer generated synthetic image, and the like. The image acquisition device may include, for example, an MR imaging device, a CT imaging device, a PET imaging device, an ultrasound imaging device, a fluoroscopic device, a SPECT imaging device, an integrated linear accelerator, an MR imaging device, and the like. Furthermore, the image acquisition device is not limited to the above-described examples and may include a variety of medical imaging devices within the scope of the technical idea for acquiring medical images of a patient.

A paired data set may include a set of training data including images of an object such as a particular part of a patient.

For example, a CT imaging device and an MR imaging device may be used to acquire a CT image and an MR image, respectively, of a particular part of a patient, such as the head, in which case the CT image and the MR image may form a paired data set.

In a case in which a non-enhanced (or non-contrast enhanced) CT image and an MR image are respectively acquired for a particular part of a patient, the non-enhanced CT image and the MR image may form a paired data set. In a case in which an enhanced (or contrast enhanced) CT image and an MR image are respectively acquired, the enhanced CT image and the MR image may form a paired data set.

In a case in which T1 and T2 images are acquired from a particular part of a patient, the T1 and T2 images may form a paired data set.

In this context, the particular part of a patient may mean the particular part of a single patient. In a case in which imaged parts are the same, the imaged parts may refer to particular parts of different patients.

For example, CT and MR images acquired from a particular part of a single patient may form a paired data set, and CT and MR images acquired from the same parts of different patients may also form a paired data set.

Different domains of two images mean that the domains are different, for example, in a case in which respective patterns contained in two matching images have different contrasts or two images have different intensity distributions or different patterns.

Furthermore, images acquired using different imaging devices belong to different domains. For example, because CT and MR images are acquired using a CT imaging device and an MR imaging device, respectively, both images belong to different domains. Similarly, there are domain differences between ultrasound and CT images, between ultrasound and MR images, and between MR and PET images.

Furthermore, non-enhanced and enhanced images have different intensity distributions for the same part, and thus belong to different domains.

For MR images, T1 and T2 images are the same MR images, but have different intensity distributions and therefore belong to different domains. That is, in a case in which two images realized by reconstruction based on signals provided from an object have a difference in signals, sequences, or image realization processes on which the image realization is based, the two images having the difference belong to different domains. Furthermore, the medical image examples described above belong to different domains.

To illustrate the method of converting a medical image according to an embodiment of the disclosure, a case in which the image acquisition device has obtained a paired data set by acquiring a first image 110 and a second image 120 from the same part of a single patient will be described as an example. A case in which the first image 110 is an original CT image, the second image 120 is an original MR image, and the third image 130 is a synthetic MR image according to embodiments will be described.

The first operation S100 may include receiving, by the learning model, the first image 110 selected from the paired data set including the first image 110 and the second image 120. The first image 110 may include an input image processed from an original CT image with training data.

In the paired data set including the first image 110 and the second image 120, which one of the first image 110 and the second image 120 is to be selected as the input image may be determined by the manufacturer of medical device software which performs the method of converting a medical image according to this embodiment. In this case, which image is to be selected may be selected automatically by a selection algorithm, or may be selected manually by an operator.

The manufacturer may acquire the original CT image from the CT imaging device and the original MR image from the MR imaging device to form a paired data set, and may select the original CT image as the input image from the paired data set by an algorithm or manually.

The second operation S100 may include constructing the third image 130 based on the first image 110, in which the learning model having received the first image 110 constructs the third image 130 which matches the first image 110 and belongs to a different domain from the first image 110.

For example, the learning model receives the original CT image as an input image and constructs a synthetic MR image based on the original CT image.

The process of the learning model constructing the third image 130 from the first image 110 which belongs to a different domain from the first image 110 may be performed by the following two processes according to embodiments. However, the process of the learning model constructing the third image 130 is not limited to the following two processes.

The first process may include one or more layers which receive the first image 110 obtained from the image acquisition device and perform convolutional operations to extract high-dimensional features having a smaller size than the first image 110, in which the layers may be represented by an artificial neural network configured to extract features. Such a feature extraction artificial neural network may receive two- or three-dimensional real image data obtained from the image acquisition device and perform convolutional operations to extract image features from the original images.

Describing this process by way of example, a convolutional layer which extracts the features of an image through a filter and a pooling layer which enhances the features and reduces the size of the image may be provided to extract the features of an image by repeating the convolution and the pooling.

In some embodiments, a convolutional layer including a plurality of layers may extract features of an input image given as an input from a first convolutional layer and produce a feature map as a result, a pooling layer may receive this feature map and produce a result having enhanced features and a reduced image size, and the output of the pooling layer may be input again into a second convolutional layer.

The second process includes one or more layers performing a deconvolution operation for the purpose of generating image data of different modalities from the output of the first process described above, in which these layers may be represented by an artificial neural network for generating images of different modalities. The artificial neural network which generates images of different modalities may perform a deconvolution operation on the result of the convolution operation of extracting the features by the artificial neural network in the first process to generate two- or three-dimensional image data of different modalities.

The third operation S120 may include operations of comparing the third image 130 with the second image 120 and training the learning model considering the comparison result. In a case in which the original CT image in the paired data set which includes the original CT image and the original MR image is determined to be the input image, the original MR image may be determined to be the target image. The second image 120 may correspond to ground truth. After constructing the third image 130, the learning model may be trained by comparing the constructed third image 130 with the second image 120 to reduce the difference between the constructed third image 130 and the second image 120. That is, the learning model may be trained by comparing the synthetic MR image with the original MR image and continuously reviewing whether the synthetic MR image is constructed to be similar to the original MR image so that the synthetic MR image and the original MR image are the same. In some embodiments, an operation of calculating a loss value between the third image 130 and the second image 120 by applying a loss function to the algorithm may be included, and the parameters of the learning model may be updated based on the loss value.

In some embodiments, in the operation of calculating a loss value, the calculation of the loss value to update the learning model may be performed at each iteration. That is, the learning model may calculate the loss value between the synthetic MR image and the original MR image at each iteration during the training.

In a case in which the iteration includes a first iteration section and a second iteration section which are sequential, in some embodiments, the learning model may recognize overfitting and terminate the training in a case in which the absolute value of the change between the first loss value calculated in the first iteration section and the second loss value calculated in the second iteration section is less than a reference value.

In a case in which the learning model is sufficiently trained about the medical image conversion, the training may no longer be performed and be completed. The model having completed the training is now able to construct medical images as third images 130 with respect to a plurality of first images 110 having different data distributions, the third images 130 belonging to different domains from the first images 110.

A learning model 200 according to an embodiment of the disclosure may use a GAN model, and may include a constructor and a discriminator. The constructor may be trained to construct images, and the discriminator may be trained to discriminate images.

Referring to FIG. 2, in the learning model 200 using the GAN model, in a case in which the original CT image 110 is input as an input image to a constructor 210, the constructor 210 may be trained to construct a synthetic MR image 130 by the convolutional operation described above, and a discriminator 220 may be trained to discriminate the synthetic MR image 130 from an original MR image 120, i.e., a real medical image.

In some embodiments, the learning model 200 according to this embodiment may use a cycle GAN model (not shown). In this case, the learning model 200 may include the first constructor 210, the first discriminator 220, a second constructor, and a second discriminator. In a case in which the original CT image 110 is input to the first constructor 210 as an input image, the first constructor 210 may be trained to construct the synthetic MR image 130 through the convolutional operation described above, and the first discriminator 220 may be trained to discriminate a synthetic MR image from the original MR image 120, i.e., the real medical image. In a case in which the synthetic MR image 130 constructed by the first constructor 210 is then input to the second constructor, the second constructor may be trained to construct a synthetic CT image based on the synthetic MR image 130, and the second discriminator may be trained to discriminate a synthetic CT image from an original CT image.

Here, the original CT image 110 may correspond to the first image 110 in the method of converting a medical image described above, the synthetic MR image 130 may correspond to the third image 130, and the original MR image 120 may correspond to the second image 120.

In some embodiments, the third operation S120 may include calculating a match rate between the third image 130 and the second image 120 and feeding the calculated value back to the learning model.

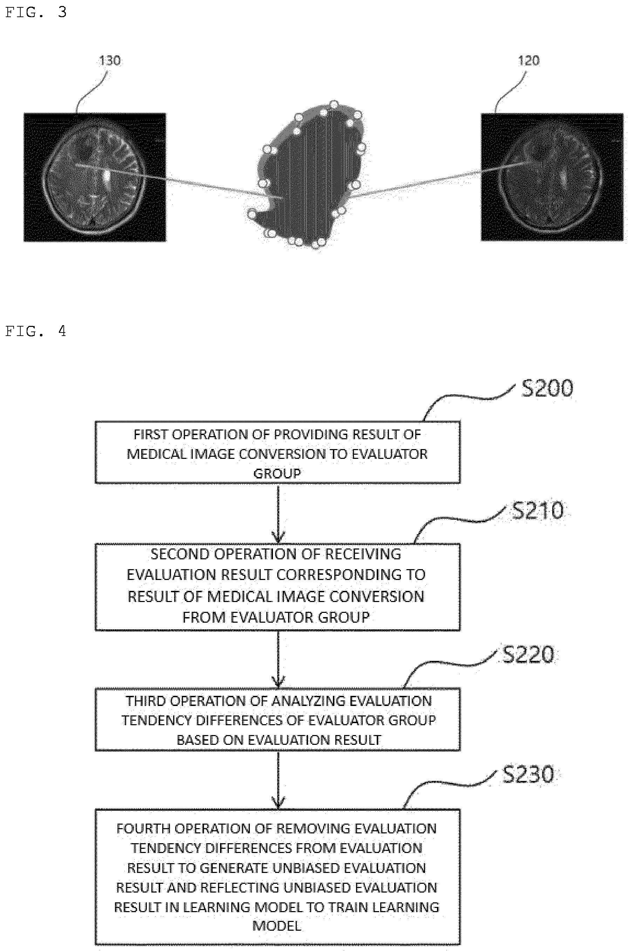

FIG. 3 illustrates an embodiment of calculating the match rate between an output image and a target image.

In some embodiments, in a case in which a lesion region is marked on each of the same parts of a synthetic MR image and an original MR image, the calculation of the match rate may be performed by comparing the sizes of the lesion regions. For example, the match rate may be calculated by marking the location of a tumor or other lesion in the synthetic MR image and the original MR image, respectively, followed by conversion to a binary image.

In this case, the match rate may be calculated using an evaluation metric, such as the Hausdorff distance and the Dice similarity coefficient, or calculated quantitatively based on the difference between the centers of mass of the two lesion locations. Furthermore, in a case in which the target image and the output image are CT images, the match rate of the two images may be determined by comparing the radiation dose calculations for the lesions. In some embodiments, the determination may be performed by representing the difference between the synthetic MR image and the original MR image using numerical values such as MAE, RMSE, SSIM, or PSNR (hereinafter referred to as a performance metric).

Referring to FIG. 3, the process of calculating the match rate by aligning the lesion regions of the synthetic MR image 130, i.e., the output image, and the original MR image 120, i.e., the target image, and comparing the contours of the respective lesions using the Hausdorff distance measure is illustrated.

FIG. 4 illustrates an Al training method according to another embodiment of the disclosure.

An Al training method according to an embodiment of the disclosure is an Al training method for improving the versatility of a learning model 200 which is trained using a generative adversarial network (GAN), receives input data and generates an output image for medical image conversion, and compares the output image with a target image to improve the accuracy of the result of the medical image conversion. The learning model 200 includes:

a first operation S200 of providing a medical image conversion result to an evaluator group (or a group of evaluators); a second operation S210 of receiving an evaluation result corresponding to the medical image conversion result from the evaluator group;

a third operation S220 of analyzing evaluation tendency differences of the evaluator group based on the evaluation result; and

a fourth operation S230 of removing the evaluation tendency differences from the evaluation result to generate an unbiased evaluation result (or an evaluation result without tendency) and reflecting the unbiased evaluation result to the learning model 200 to train the learning model 200.

Here, the evaluator may generally include an expert. In some embodiments, the evaluator may be a medical professional, such as a doctor, nurse, or the like.

The evaluator group may include a group of experts who perform evaluations of medical image conversion results, and may include experts registered on a manageable platform or the like. The platform may include a website or an application which performs functions capable of providing medical image conversion results to evaluators registered on the platform and receiving comments on the conversion results from evaluators.

In the second operation S210, the medical image conversion result may include an output image output by the learning model 200. The learning model 200 performs machine learning, and after the training is completed, the learning model 200 may be provided to hospitals and the like as a software medical device to function as an assistant program for medical practice in respective hospitals.

The evaluation result from the evaluator group may include an evaluation result of the resolution, quality, and the like of an output image output by the learning model 200. In general, the quality of the medical image may be scored.

In some embodiments, the third operation S220 may include analyzing a first evaluation tendency difference between different affiliated institutions and analyzing a second evaluation tendency difference between different evaluators. The affiliated institutions may include hospitals, health insurance review and assessment institutions, and the like.

The first evaluation tendency difference may include, for example, the perception of medical image conversion software between different institutions, i.e., hospital A and hospital B.

In a case in which doctor AA of hospital A and doctor BB of hospital B evaluate the same image that is the result of an image conversion device, the perception of the medical image conversion software program at hospital A may differ from the perception of the medical image conversion software program at hospital B. Hospital A may have a favorable perception of the medical image conversion software according to this embodiment and give a generous score, while hospital B may have a poor reputation of the medical image conversion software according to this embodiment and give an unusually low score. This difference may be the first evaluation tendency difference.

The second evaluation tendency difference represents an evaluation tendency difference between different evaluators.

In some embodiments, different evaluators may belong to a single institution. That is, both doctor AA and doctor BB may be doctors at hospital A. Even in a case in which the atmosphere, policies, and the like of hospital A are the same, the evaluations may differ depending on personal preference. That is, doctor AA may give relatively generous evaluations, while doctor BB may give relatively harsh evaluations.

In some embodiments, the third operation S220 may analyze the evaluation tendency differences by a statistical method. In some embodiments, the third operation S220 may analyze the evaluation tendency differences collected over a selected period of time or a selected number of times.

Because the evaluation tendency differences include subjective opinions, statistical analysis is desirable. That is, the evaluation tendency differences of doctors may be identified by analyzing a large number of evaluation results. In some embodiments, a selected period of time can may set, and the evaluation tendency differences may be analyzed based on the evaluation results submitted during this period. In other embodiments, the evaluation tendency differences collected a selected number of times may be analyzed.

The second evaluation tendency difference may be analyzed based on identification information of each evaluator, and after the score for the quality of the image is collected for a selected period of time, the evaluation tendency differences may be extracted and analyzed. The evaluation tendency differences analyzed and derived may be removed from the evaluation result and reflected as a score, which is then fed back to the Al learning model 200.

The Al learning model 200 may improve the accuracy of the output image by receiving the evaluation result of the evaluator as feedback and reflecting the evaluation result to the training. However, in this case, in a case in which the evaluation result is subjective or biased, it is not helpful to improve the accuracy of the output image. In general cases, a medical image conversion software which is trained by reflecting subjective evaluation results provided by a particular hospital is inappropriate as software used in all hospitals. Therefore, by removing the evaluation tendency differences from the evaluation result so as not to be reflected as a subjective or intolerant evaluation result, the feedback to the learning model 200 may be provided based on an unbiased evaluation result having an objective content.

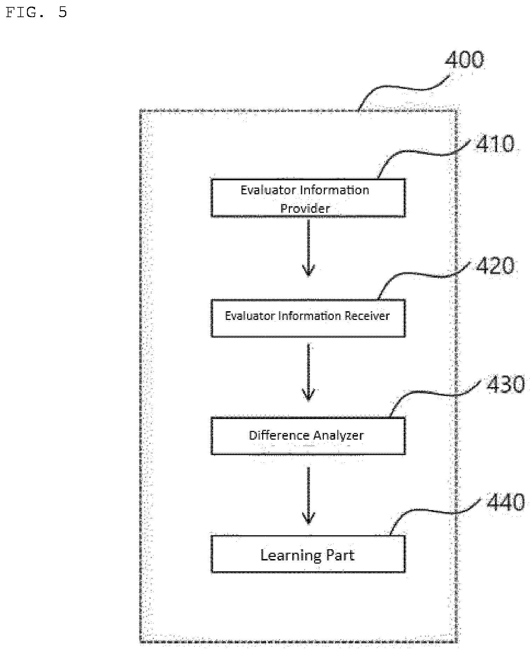

FIG. 5 illustrates a device for converting a medical image according to another embodiment of the disclosure.

A device 400 for converting a medical image according to another embodiment of the disclosure is a device 400 for converting a medical image which is trained using a generative adversarial network (GAN), and is configured to receive input data and generate an output image for medical image conversion. The device 400 includes:
an evaluator information provider 410 configured to provide a medical image conversion result to an evaluator group;
an evaluator information receiver 420 configured to receive an evaluation result corresponding to the medical image conversion result from the evaluator group;
a difference analyzer 430 configured to analyze evaluation tendency differences of the evaluator group based on the evaluation result; and
a learning part 440 configured to remove the evaluation tendency differences from the evaluation result to generate an unbiased evaluation result and reflect the unbiased evaluation result to a learning model to train the learning model.

Here, the difference analyzer 430 may include a first difference analyzer configured to analyze a first evaluation tendency difference between different institutions and a second difference analyzer configured to analyze a second evaluation tendency difference between different evaluators. In some embodiments, the different evaluators may be configured to belong to a single institution.

In some embodiments, the difference analyzer 430 may analyze the evaluation tendency differences by a statistical method. In some embodiments, the difference analyzer 430 may analyze the evaluation tendency differences collected over a selected period of time or a selected number of times.

The device 400 for converting a medical image of this embodiment may be substantially the same model as the model performing the Al training method of the above-described embodiments.

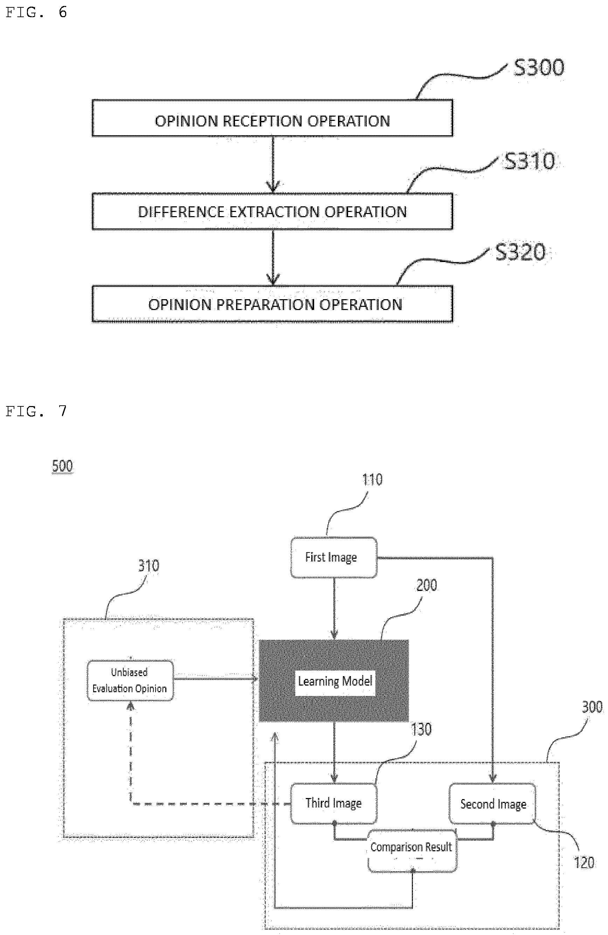

FIG. 6 illustrates a method of preprocessing training data according to an embodiment of the disclosure.

In another aspect of the disclosure,

A method of improving a conversion result of a learning model which is trained using a generative adversarial network (GAN) and performs a medical image conversion may be provided. The method includes:
an opinion reception operation S300 of providing a medical image conversion result to an evaluator group and receiving evaluation opinions about the medical image conversion result from the evaluator group;
a difference extraction operation S310 of extracting evaluation tendency differences of the evaluator group based on the evaluation opinions; and
an opinion preparation operation S320 of removing the extracted evaluation tendency differences from the evaluation opinions to prepare an unbiased evaluation opinion from which the evaluation tendency differences are removed.

FIG. 7 illustrates a system for converting a medical image according to an embodiment of the disclosure.

A system 500 for converting a medical image according to an embodiment of the disclosure may include: a learning model 200 configured to receive a first image 110 and construct a third image 130 belonging to a different domain from the first image 110 based on the first image 110;
a first feedback part 300 configured to compare the third image 130 with a second image 120 and feed a comparison result back to the learning model 200; and
a second feedback part 310 configured to provide the third image 130 to evaluators, receive evaluation opinions of the evaluators, extract evaluation tendency differences of the evaluators, remove the evaluation tendency differences from the evaluation opinions to derive an unbiased evaluation opinion from which the evaluation tendency differences are removed, and feed the unbiased evaluation opinion back to the learning model 200.

Here, the first image 110, the second image 130, and the third image 120 shown in the drawings may correspond to the input image, the target image, and the output image, respectively, of the above-described embodiments.

The above-described embodiments of the disclosure may be implemented in the form of computer programs executable on a computer using various components, and such computer programs may be stored in computer-readable media. Examples of the computer-readable media may include: magnetic media such as hard disks, floppy disks, and magnetic tapes; optical recording media such as CD-ROMs and DVDs; magneto-optical media such as floptical disks; and hardware devices such as ROMs, RAMs, and flash memories specifically configured to store program instructions and execute the program instructions.

Furthermore, the computer programs may be specifically designed and configured for the disclosure or may be known and available to a person having ordinary knowledge in the art of computer software. Examples of computer programs may include machine code produced by compilers and high-level language code executable on computers using interpreters.

In the specification (in particular Claims) of the disclosure, the use of the term "the" and similar denoting terms may correspond to both singular and plural forms. Furthermore, recitation of ranges of values herein are intended merely to refer to respective separate values falling within the respective ranges and, unless otherwise indicated herein, the respective separate values are incorporated herein as if individually recited herein.

Finally, the operations of any method described herein may be performed in any suitable order unless otherwise indicated herein or otherwise clearly contradicted by the context. However, the operations shall not be limited to the described sequence. The use of any examples or illustrative languages (e.g., "such as") provided herein, is intended merely to better illustrate the disclosure and does not pose a limitation on the scope of the disclosure unless otherwise defined by the Claims. Furthermore, a person having ordinary knowledge in the art will appreciate that various modifications, combinations, and changes are possible according to design conditions and factors within the scope of the Claims or equivalents thereof.

Therefore, the spirit of the disclosure shall not be limited to the above-described embodiments, and the entire scope of the appended claims and equivalents thereof will fall within the scope and spirit of the disclosure.

### <Description of Reference Numerals of Drawings>

- 110:: first image
- 120:: second image
- 130:: third image
- 200:: learning model
- 300:: first feedback part
- 310:: second feedback part
- 400:: device for converting a medical image
- 410:: evaluator information provider
- 420:: evaluator information receiver
- 430:: difference analyzer
- 440:: learning part
- 500:: system for converting a medical image

## Claims

1. An artificial intelligence training method for improving versatility of a learning model which is trained using a generative adversarial network (GAN), receives input data and generates an output image for medical image conversion, and compares the output image with a target image to improve accuracy of a result of the medical image conversion, the artificial intelligence training method comprising:
a first operation of providing a result of medical image conversion to an evaluator group;
a second operation of receiving an evaluation result corresponding to the result of the medical image conversion from the evaluator group;
a third operation of analyzing evaluation tendency differences of the evaluator group based on the evaluation result; and
a fourth operation of removing the evaluation tendency differences from the evaluation result to generate an unbiased evaluation result and reflecting the unbiased evaluation result in the learning model to train the learning model.

2. The artificial intelligence training method of claim 1, wherein the third operation comprises analyzing a first evaluation tendency difference between different affiliated institutions and analyzing a second evaluation tendency difference between different evaluators.

3. The artificial intelligence training method of claim 2, wherein the different evaluators belong to a single institution.

4. The artificial intelligence training method of claim 1, wherein the third operation analyzes the evaluation tendency differences by a statistical method.

5. The artificial intelligence training method of claim 4, wherein the third operation analyzes the evaluation tendency differences collected over a selected period of time or a selected number of times.

6. A device for converting a medical image which is trained using a generative adversarial network (GAN), and receives input data and generates an output image for medical image conversion, the device comprising:
an evaluator information provider configured to provide a result of medical image conversion to an evaluator group;
an evaluator information receiver configured to receive an evaluation result corresponding to the result of the medical image conversion from the evaluator group;
a difference analyzer configured to analyze evaluation tendency differences of the evaluator group based on the evaluation result; and
a learning part configured to remove the evaluation tendency differences from the evaluation result to generate an unbiased evaluation result and reflect the unbiased evaluation result to a learning model to train the learning model.

7. The device of claim 6, wherein the difference analyzer comprises a first difference analyzer configured to analyze a first evaluation tendency difference between different institutions and a second difference analyzer configured to analyze a second evaluation tendency difference between different evaluators.

8. The device of claim 7, wherein the different evaluators belong to a single institution.

9. The device of claim 6, wherein the difference analyzer analyzes the evaluation tendency differences by a statistical method.

10. The device of claim 9, wherein the difference analyzer analyzes the evaluation tendency differences collected over a selected period of time or a selected number of times.

11. A method of improving a conversion result of a learning model which is trained using a generative adversarial network (GAN) and performs a medical image conversion, the method comprising:
an opinion reception operation of providing a result of medical image conversion to an evaluator group and receiving evaluation opinions about the result of the medical image conversion from the evaluator group;
a difference extraction operation of extracting evaluation tendency differences of the evaluator group based on the evaluation opinions; and
an opinion preparation operation of removing the extracted evaluation tendency differences from the evaluation opinions to prepare an unbiased evaluation opinion from which the evaluation tendency differences are removed.

12. A system for converting a medical image, the system comprising:
a learning model configured to receive an input image and generate an output image belonging to a different domain from the input image based on the input image;
a first feedback part configured to compare the output image with a target image and feed a result of the comparison back to the learning model; and
a second feedback part configured to provide the output image to evaluators, receive evaluation opinions of the evaluators, extract evaluation tendency differences of the evaluators, remove the evaluation tendency differences from the evaluation opinions to derive an unbiased evaluation opinion from which the evaluation tendency differences are removed, and feed the unbiased evaluation opinion back to the learning model.
